(19) Europäisches Patentamt
European Patent Office
Office européen des brevets

(11) **EP 4 249 500 A2**

(12) **EUROPEAN PATENT APPLICATION**
published in accordance with Art. 153(4) EPC

(43) Date of publication:
**27.09.2023 Bulletin 2023/39**

(21) Application number: **21865361.6**

(22) Date of filing: **18.11.2021**

(51) International Patent Classification (IPC):
**C07K 14/435** $^{(2006.01)}$    **A61K 38/17** $^{(2006.01)}$

(52) Cooperative Patent Classification (CPC):
(C-Sets available)
**C07K 14/43509; A61K 31/407; A61K 38/1767;**
**A61K 45/06;** Y02A 50/30          (Cont.)

(86) International application number:
**PCT/CU2021/050011**

(87) International publication number:
**WO 2022/105948 (27.05.2022 Gazette 2022/21)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **19.11.2020  CU 20200086**

(71) Applicant: **Centro de Investigación y Desarrollo de**
**Medicamentos CIDEM**
**10400 la Habana (CU)**

(72) Inventors:
• **MONTERO-ALEJO, Vivian**
  **10700 La Habana (CU)**
• **PERDOMO-MORALES, Rolando**
  **10700 La Habana (CU)**
• **VÁZQUEZ-GONZÁLEZ, Amanda**
  **17100  La Habana (CU)**
• **GARAY-PEREZ, Hilda Elisa**
  **10600  La Habana (CU)**

(74) Representative: **V.O.**
  **P.O. Box 87930**
  **2508 DH Den Haag (NL)**

Remarks:
The complete document including Reference
Table(s) and the Sequence Listing(s) can be
downloaded from the EPO website

(54) **PEPTIDE ENTITIES WITH ANTIMICROBIAL ACTIVITY AGAINST MULTI-DRUG RESISTANT PATHOGENS**

(57)    The present invention describes synthetic peptide compounds that combat pathogenic microorganisms of a bacterial nature, and that appear as microorganisms that are multi-resistant to conventional antibiotics. It further describes combination therapies containing said peptides. Pharmaceutical compositions comprising peptide compounds and their combinations are described; as well as the uses and treatment methods in which they can be used.

EP 4 249 500 A2

(52) Cooperative Patent Classification (CPC): (Cont.)

C-Sets
**A61K 31/407, A61K 2300/00;**
**A61K 38/1767, A61K 2300/00**

**EP 4 249 500 A2**

**Description**

Technical field:

The present invention relates to new peptide entities, combinations, and pharmaceutical compositions comprising them; as well as their use in the manufacture of medicines and in methods of treating bacterial infections.

Background of the invention:

Antibiotic resistance occurs when germs like bacteria and fungi develop the ability to defeat or circumvent drugs designed to kill them. That means that the germs do not die and continue to grow. Infections caused by germs resistant to antibiotics are difficult and sometimes impossible, to treat. If we add to this that in most cases antibiotic-resistant infections require long hospital stays, additional medical follow-up visits, and expensive and toxic treatment alternatives, the complexity of the problem is even greater. Antibiotic resistance does not mean that the body is becoming resistant to antibiotics as occurs in resistance phenomena in other diseases; rather, the bacteria have become resistant to antibiotics designed to kill them. This problem is not solved by increasing the dose of antibiotics to be administered, which also represents an added risk of toxicity. Antibiotic resistance is capable of affecting people at any stage of life, as well as the health, veterinary and agricultural industries, making it one of the most serious and urgent health problems in the world. Although the risk of resistant infections cannot be completely avoided, some people are at higher risk than others, for example, those with chronic diseases. If antibiotics lose their effectiveness, we lose the ability to treat infections and control threats to public health (https://www.cdc.gov/drugresistance/intl-activities/amr-challenge.html). In addition, in most of the various non-infectious disease scenarios, clinicians also rely on the ability to prevent infections through the use of antibiotics, for example in joint replacements, organ transplants, antiinflammatory therapy, cancer and in the treatment of chronic diseases such as diabetes, asthma, and rheumatoid arthritis.

Great efforts have been made to use antibiotics in combination with adjuvants targeting important metabolic pathways and mechanisms, which contribute to resistance to conventional antibiotics (permeabilizers, lactamase inhibitors, efflux pump inhibitors, quorum sensing inhibitors, inhibitors of toxins, etc.) The modest perceived success to date with such combinations of antibiotics and adjuvants has paved the way for exploring other alternatives to combat resistance to these drugs.

Since the beginning of 2017, the World Health Organization (WHO) publishes a list of priority pathogens worldwide containing 12 drug-resistant bacteria, for which new antibiotics are urgently required. This report stresses the importance and pressing need to develop new antibiotics against multidrug-resistant gram-negative bacteria. (see https://www.who.int/news-room/detail/27-02-2017-who-publishes-list-of-bacteria-for-which-new-antibiotics-are-urgently-needed). Among the microorganisms that are reported with the highest incidence of antibiotic resistance are *Acinetobacter baumannii* sp, resistant to carbapenems, *Pseudomonas aeruginosa* sp, resistant to carbapenems and *Enterobacteriaceae* spp, resistant to carbapenems and producing ESBL. In addition, other microorganisms with an intermediate incidence are mentioned, such as *Enterococcus faecium* sp, resistant to vancomycin, and *Staphylococcus aureus* sp, resistant to methicillin.

The ability of antimicrobial peptides (AMPs) to interact with the bacterial membrane and cause cell lysis makes them a promising alternative to combat the phenomenon of resistance of pathogens to conventional antibiotics [Mulani, MS, et al., Emerging Strategies to Combat ESKAPE Pathogens in the Era of Antimicrobial Resistance: A Review. Frontiers in microbiology, 2019. 10: p. 539-539]. The membrane lytic mechanism of natural AMPs is a potentially promising therapeutic alternative because resistance mechanisms rapidly emerge to specific drug targets [Mourtado, R., et al., Design of stapled antimicrobial peptides that are stable, non-toxic and kill antibiotic-resistant bacteria in mice. Nature Biotechnology, 2019. 37 (10): p. 1186-1197]. In this sense, today the need for new external anti-infective agents is more urgent to combat infectious diseases, and specifically, those unresolved and/or caused by multi-drug-resistant microorganisms.

As a general rule, AMPs are selective towards negatively charged bacterial membranes such as bacteria, and their cytotoxicity is moderate towards eukaryotic organisms which present a neutral charge in their surface. A distinctive element in the antibacterial mechanism of AMPs is their interaction with the cytoplasmic membrane, therefore charge and hydrophobicity are key properties for the development of the antimicrobial activity. Once the peptide-membrane binding has occurred, the mechanism of action generally involves the formation of lytic pores or destabilization of the membrane by the formation of peptide aggregates. Other non-lytic mechanisms include cell depolarization, and translocation to the cytosol, and binding to intracellular targets (nucleic acids, enzymes, etc.) [Hale, J.D. and R.E. Hancock, Alternative mechanisms of action of cationic antimicrobial peptides on bacteria. Expert Rev Anti Infect Ther, 2007. 5 (6): p. 951-9.].

There is no evidence of therapeutic candidates from natural templates from the hemolymph of crustaceans of the genus *Panulirus* ssp. The presence of a new family of β-type antimicrobial peptides defensins was recently described in hemocytes of the spiny lobster *Panulirus argus* [Montero-Alejo, V., et al., Defensin-like peptide from Panulirus argus relates structurally with beta-defensin from vertebrates. Fish Shellfish Immunol, 2012. 33 (4): p. 872-9.] [Montero-Alejo, V., et al., Panusin represents a new family of beta-defensin-like peptides in invertebrates. Dev Comp Immunol, 2017. 67: p. 310-321]. A new family of trypsin-type protease inhibitors that regulate the prophenoloxidase activating system was also identified in this crustacean [Perdomo-Morales, R., et al., The trypsin inhibitor panulirin regulates the prophenoloxidase-activating system in the spiny lobster Panulirus argus. J Biol Chem, 2013. 288 (44): p. 31867-79] [WO / 2013/113296. PERDOMO, M.R., et al., Composition from lobster hemocyte extract for detection of lipopolysaccharides, peptidoglycans, and 1,3-beta-d-glucans. 2014.] As mentioned above, the phenomenon of Multi-drug-resistant (MDR) pathogens and the lack of new therapeutic alternatives that allow us to face this problem makes it necessary to search for new molecules with antimicrobial activity and novel mechanisms of action.

BRIEF DESCRIPTION OF THE INVENTION

The present invention provides synthetic peptide sequences from the group comprising the sequences SEQ ID NO. 1, SEQ ID NO. 2, and SEQ ID NO. 3 with potential therapeutic applications in the prevention and treatment of microbial infections caused by multi-drug resistant bacteria.

The invention further provides a combination therapy to a patient with a bacterial infection to control the development of the multi-resistance phenomenon, which comprises the administration to the patient of at least 2 sequences selected from the group comprising SEQ ID NO. 1-3. And it also provides a combination therapy comprising the administration to the patient of a sequence selected from the group comprising SEQ ID NO. 1-3 with a beta-lactam antibiotic. And more specifically it provides this combination therapy where the beta-lactam antibiotic class are carbapenems.

Another object of the present invention is a pharmaceutical composition comprising a peptide selected from the group consisting of SEQ ID NO. 1-3 as an active ingredient and one or more pharmaceutically acceptable excipients. Another object of the invention is a pharmaceutical composition that further comprises a second peptide selected from the group consisting of SEQ ID NO. 1-3 as an active ingredient. Another object of the invention is a pharmaceutical composition comprising a peptide selected from the group consisting of SEQ ID NO. 1-3 and also a beta-lactam antibiotic. A particular embodiment of this object is where the antibiotic is meropenem.

The peptides described in the present invention can be used for the manufacture of drugs for the treatment of bacterial infections. A distinctive aspect of the present invention is its use in the treatment of infections caused particularly by Gram-negative bacteria. Particular forms of carrying out the invention are its therapeutic use in the treatment of infections caused by pathogenic strains belonging to the ESKAPE group, for example, *Klebsiella pneumoneae* sp, *Acinetobacter baumannii* sp, *Pseudomona aeruginosa* sp. and *Escherichia coli* sp, and more specifically, in clinical isolates of said species multi-resistant to conventional antibiotics.

Another particular aspect of the present invention is its use in the treatment of infections caused by Gram-positive bacteria. Particular forms of carrying out the invention would be its use in the treatment of infections caused by pathogenic strains selected from the group of *Enterococcus faecium* sp or *Staphylococcus aureus* sp, and more specifically, in clinical isolates of said species multi-resistant to conventional antibiotics.

Another object of the present invention is a method of treatment or preventing of a microbial infection that comprises the administration of a peptide selected from the group consisting of SEQ ID No. 1-3, of any of the combinations or the pharmaceutical compositions. A particular embodiment is where the infection is caused by a pathogenic strain belonging to the ESKAPE group.

BRIEF DESCRIPTION OF THE FIGURES

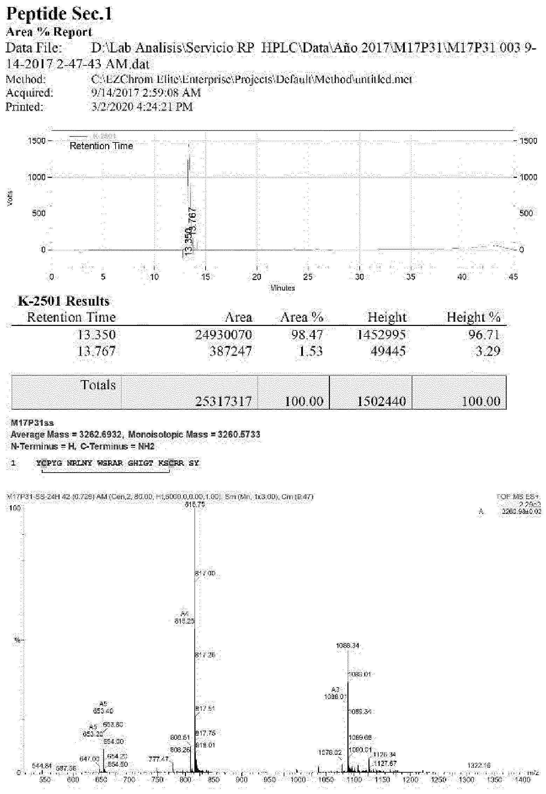

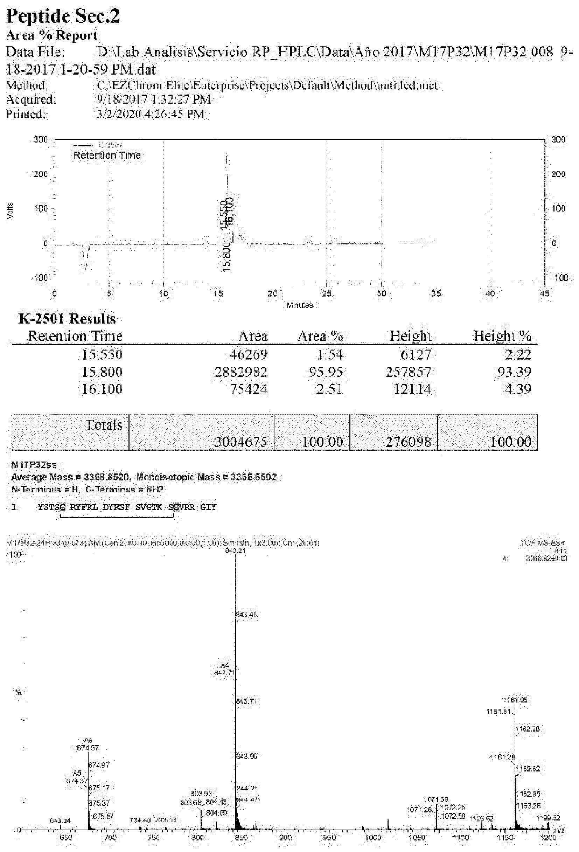

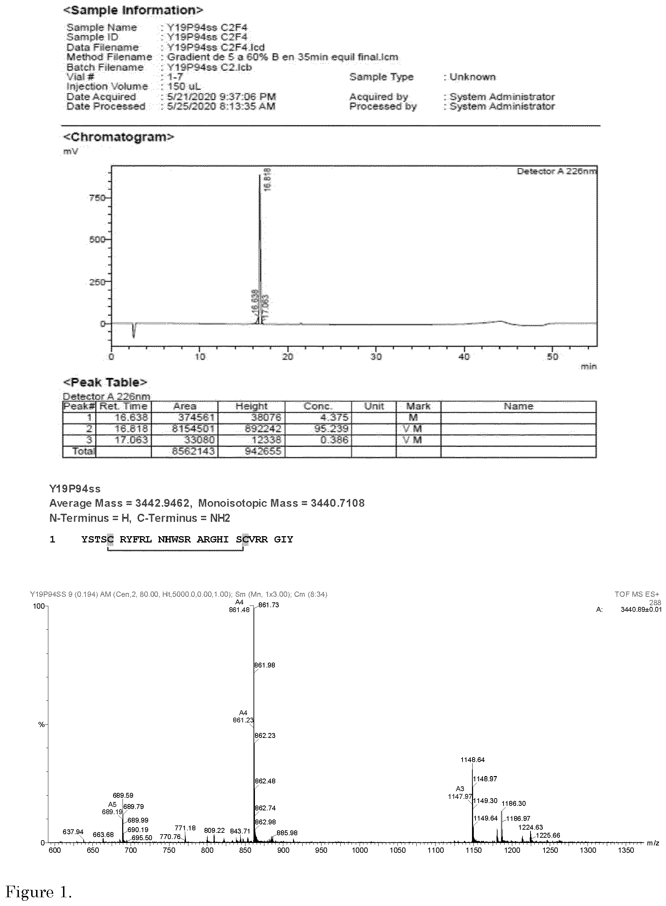

Figure 1. Purification by RP-HPLC and sequence check by ESI-MS / MS: a) SEQ ID NO. 1; b) SEQ ID NO. 2; c) SEQ ID NO. 3.

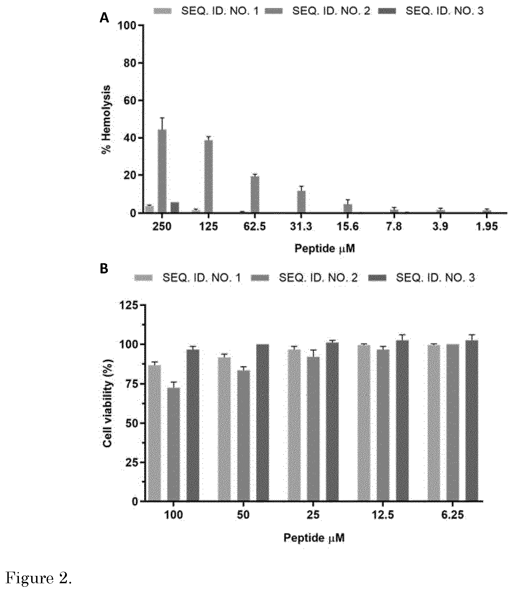

Figure 2. Evaluation of the toxic effects of peptides SEQ ID NO. 1, SEQ ID NO. 2, and SEQ ID NO. 3 on different cell types. A) Percentage of hemolysis determined in vitro at different peptide concentrations on erythrocytes of Wistar rats. B) Percentage of cell viability determined in vitro at different peptide concentrations on the Hep2 cell line. The peptide concentrations were used to cover a value up to 10 times higher than the 90% lethal concentration ($LD_{90}$) values of bacterial growth.

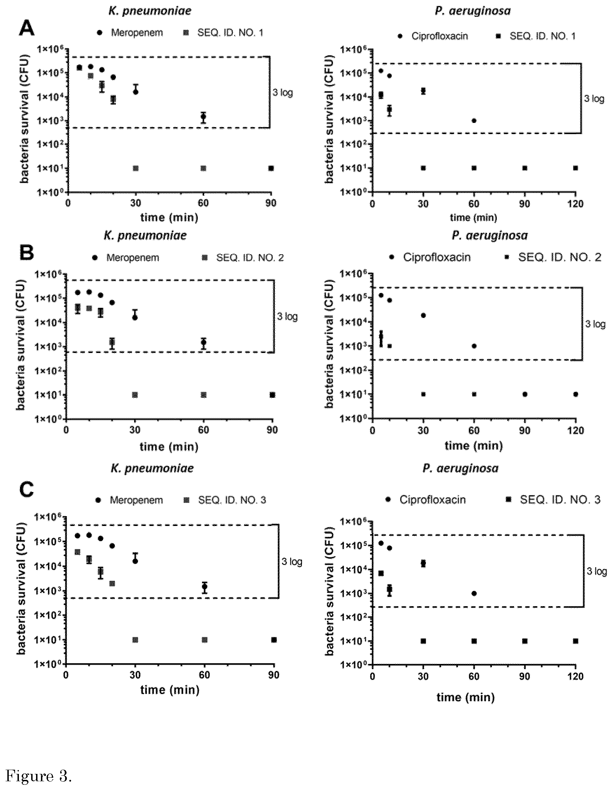

Figure 3. Evaluation of the death time of the bacteria *Klebsiella pneumoniae* and *Pseudomona aeruginosa:* A) SEQ ID NO. 1, B) SEQ ID NO. 2, C) SEQ ID NO. 3. In each case, the antibiotic meropenem was used to compare the death time of *K. pneumoniae* and the antibiotic ciprofloxacin for the *P. aeruginosa* bacterium. A 3-log reduction in the number of colonies in the time evaluated demonstrates the lethal effect on the selected bacteria.

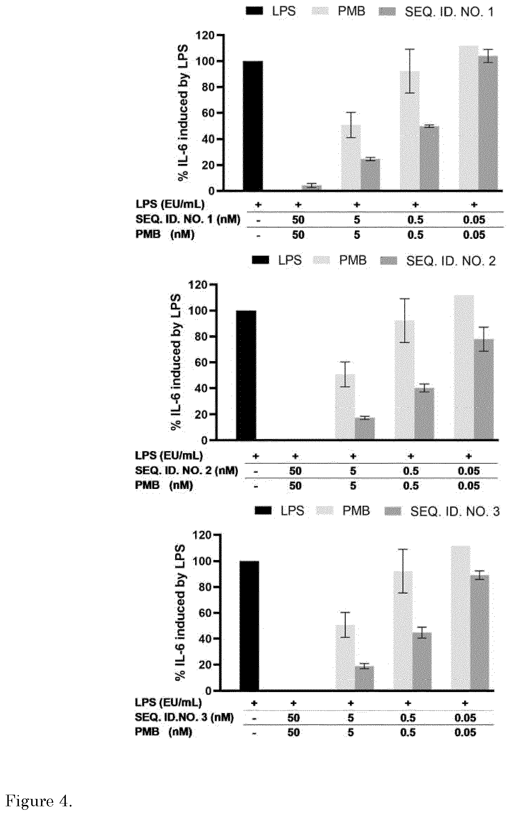

Figure 4. Inhibition of LPS-induced IL-6 release from whole human blood culture in the presence of the peptide sequences: A) SEQ ID NO. 1, B) SEQ ID NO. 2, C) SEQ ID NO. 3, and comparison with the LPS neutralizing activity of polymyxin B (PMB) as a reference.

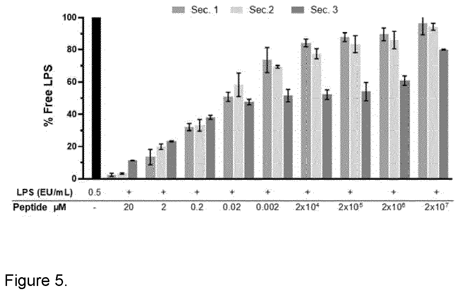

Figure 5. LPS neutralizing activity of peptides determined by the Limulus Amebocyte Lysate (LAL) method. Different concentrations of peptides were incubated with LPS (0.5 EU / ml). The LAL method allows us to determine the presence of active LPS (recovery percentage) in the presence of peptides. Dose-dependent inhibition of the response of the LAL assay is obtained against the LPS challenge, in a concentration range of 2 nM-20 $\mu$M for each peptide variant.

DETAILED DESCRIPTION OF THE INVENTION

The present invention provides an alternative based on new synthetic peptide compounds that combat pathogenic microorganisms of a bacterial nature, and that appears as multiresistant microorganisms to conventional antibiotics. Surprisingly, the chimeric hybrid structures designed from different families of natural peptides identified in the hemocytes of the lobster *P. argus,* show antimicrobial activity against multiresistant antibiotic strains that include *Enterococcus faecium* sp., *Staphylococcus aureus* sp., *Klebsiella pneumoneae* sp., *Acinetobacter baumannii* sp., *Pseudomonas aeruginosa* sp. and *Eccherichia coli* sp.

In a preferred embodiment of the present invention, the list of amino acid sequences that are selected from the group consisting of the sequences SEQ ID NO is shown. 1; SEQ ID NO. 2; and/or SEQ ID NO. 3, or a peptide structure with at least 80% identity with SEQ ID NO. 1-3 is shown.

In a preferred embodiment, the selected sequences comprised in the group SEQ ID NO. 1; SEQ ID NO. 2; and/or SEQ ID NO. 3 are obtained by chemical synthesis using the solid phase peptide synthesis (SPPS) methodology. That said, in the primary structure of these sequences there are at least 6 basic amino acids corresponding to arginine or lysine that can occur in their L or D forms, and in a preferred embodiment the sequences obtained must contain at least four residues of arginine.

The present invention describes a combination therapy that includes at least one of the selected sequences comprised in the group SEQ ID NO. 1; SEQ ID NO. 2; and/or SEQ ID NO. 3. In said combination therapy, the peptides can be found in the same formulation or in different formulations that can be administered concomitantly or sequentially.

In another embodiment, the present invention provides a pharmaceutical composition comprising at least one synthetic peptide selected from the group consisting of SEQ ID NO. 1; SEQ ID NO. 2; and/or SEQ ID NO. 3 as an active ingredient and one or more pharmaceutically acceptable excipients.

In another embodiment, the present invention provides the use of the sequences SEQ ID NO. 1; SEQ ID NO. 2; and/or SEQ ID NO. 3 in the prevention and/or treatment of microbial infections in a subject. The peptide sequences of the present invention are broad-spectrum antibacterial because they have a lytic effect on Gram-negative and Gram-positive bacterial strains, which cause infections in humans and/or animals.

In a preferred embodiment, the present invention provides the use of said peptide sequences SEQ ID NO. 1; SEQ ID NO. 2; and/or SEQ ID NO. 3 are inhibitors of bacterial growth and with bactericidal activity against selected pathogenic microorganisms from the group *Enterococcus faecium* sp, *Staphylococcus aureus* sp, *Klebsiella pneumoneae* sp, *Acinetobacter baumannii* sp, *Pseudomonas aeruginosa* sp and *Eccherichia coli* sp.

In a preferred embodiment, the present invention provides the use of peptide sequences SEQ ID NO. 1; SEQ ID NO. 2; and/or SEQ ID NO. 3 as growth inhibitors of multi-resistant pathogenic microorganisms such as *Enterococcus faecium* sp, *Staphylococcus aureus* sp, *Klebsiella pneumoneae* sp, *Acinetobacter baumannii* sp, *Pseudomonas aeruginosa* sp, and *Eccherichia coli* sp. Consequently, all antibiotic multi-resistant microorganisms were sensitive to peptide compounds, presenting $CL_{90}$ values between 1.0 -8.0 $\mu$M in the kinetic microdilution method.

In another embodiment, the present invention provides the use of peptide sequences SEQ ID NO. 1; SEQ ID NO. 2; and/or SEQ ID NO. 3 to obtain a 3-log decrease in the initial bacterial load in times less than 30 minutes. It is a fact that these provide rapidity to achieve the reduction of the bacterial load compared to the antibiotics meropenem and ciprofloxacin against the selected bacteria *Klebsiella pneumoniae* (ATCC 1003) and *Pseudomona aeruginosa* (ATCC 9027).

In a preferred embodiment, it is exemplified that the peptide sequences SEQ ID NO. 1; SEQ ID NO. 2; and/or SEQ ID NO. 3 show neutralizing activity of lipopolysaccharide or LPS, the main and toxic component of the outer membrane of Gram-negative bacteria. Preferably, the peptide sequences inhibit LPS-induced pro-inflammatory cytokine release in whole blood culture and inhibit the LPS response to the LAL (Limulus amebocyte lysate) reagent.

The invention further provides an embodiment that exemplifies the synergistic antibacterial effect obtained from the combinations of SEQ ID NO. 1- SEQ ID NO. 2; SEQ ID NO. 1- SEQ ID NO. 3 and SEQ ID NO. 2- SEQ ID NO. 3 in an *in vitro* culture of multi-antibiotic resistant strains of *Klebsiella pneumoniae* sp and *Staphylococcus aureus* sp from clinical isolates.

The present invention further provides a method of treatment of a subject diagnosed with a microbial infection, which comprises the administration of a therapeutically effective amount of one of the peptides selected from the group formed by SEQ ID NO. 1; SEQ ID NO. 2; and/or SEQ ID NO. 3. In a preferred embodiment, said infection is an infection caused by Gram-negative and Gram-positive bacteria multi-resistant to conventional antibiotics. The present invention further provides that said treatment method guarantees a faster antibacterial response when the selected peptides of the sequences SEQ ID NO are combined. 1; SEQ ID NO. 2; and/or SEQ ID NO. 3, with the carbapenem antibiotic in an in vitro culture of *Klebsiella pneumoniae* sp and *Staphylococcus aureus* sp strains from the clinic.

EXAMPLES

Example 1.

Synthesis of peptide entities.

For peptide synthesis, Chemmatrix resin (substitution of 0.7 mmol / g) functionalized with the Fmoc-Am-OH spacer was used. The following were used as protecting groups for the side chains of amino acids: Trityl (Trt) for Cys, Asn, His, and Gln; 2,2,4,6,7-pentamethyl-dihydro benzofuran-5-sulfonyl (Pbf) for Arg; Boc for Lys; tBu for Ser, Thr, Asp, Tyr, and Glu. The Fmoc group protecting the $N\alpha$-the amino group was removed by treatment with a 20% solution of piperidine in DMF ($2 \times 10$ min). After each of the deprotection steps, it was washed 4 times with DMF for 5 minutes. The coupling of each amino acid and the spacer was carried out by the activation method with DIC / Oxyma (ethyl-2-cyano-2-(hydroximino) acetate). Four equivalents of the reagents and DMF were used as the solvent. The completion of the coupling reaction was verified by the ninhydrin method that is sensitive to the presence of free amino groups [Kaiser, E., et al., Color test for detection of free terminal amino groups in the solid-phase synthesis of peptides. Anal Biochem, 1970. 34 (2): p. 595-8]. All washings and reactions were performed with mechanical agitation and solvents or reagents were removed by vacuum filtration. The deprotection of the amino acid side chains and the separation of the peptide from the resin was carried out by treatment with a solution of TFA / H2O / EDT / TIS (94 / 2.5 / 2.5 / 1) (for peptides containing Cys or Met), for two hours at 25 °C. The unanchored peptide dissolved in TFA was precipitated in ether cooled to -20 ° C, redissolved in a 40% acetonitrile solution in water, and lyophilized. Subsequently, the sequence was verified through the processing described by González et al [Gonzalez, J., et al., Effect of the position of a basic amino acid on C-terminal rearrangement of protonated peptides upon collision-induced dissociation. J Mass Spectrom, 1996. 31 (2): p. 150-8.] ESI-MS spectra were obtained on Q-Tof 1 or Q-Tof 2 orthogonal hybrid configuration spectrometers (Micromass, England) with a nanospray ionization source. A sodium and cesium iodide solution was used as a reference for the calibration of the spectrometer. The Masslynx version 3.5 program (Micromass, England) was used for the processing of the mass spectra. Finally, the preparative purification of the peptides corresponding to the designed sequence was carried out. Preparative RP-HPLC purification was performed on LabChrom equipment (Merck Hitachi, Germany). A column of RP-C18 (Vydac, $25 \times 250$ mm, 25 $\mu$m) was used and the separation was carried out from a linear gradient of 15 to 45% of the mobile phase B during 50 min, at a flow of 5 mL/min. A composition of mobile phase A: 0.1% TFA in water (v / v) and mobile phase B: 0.05% TFA in acetonitrile (v / v) was used. 50 mg of each crude peptide was injected. Chromatograms were acquired at a wavelength of 226 nm.

Example 2.

Evaluation of the antimicrobial effect in vitro against MDR

From clinical bacterial isolates of *Enterococcus faecium, Staphylococcus aureus, Klebsiellapneumoneae, Acinetobacter baumannii, Pseudomonas aeruginosa,* and *Escherichia coli,* on LB agar wedges, a hoe was seeded in Mueller-Hinton liquid medium (MH) and allowed to grow at 37 °C. with orbital shaking at 180 rpm overnight. Subsequently, a working culture was prepared by inoculating 100 $\mu$L of growth in 5 mL of MH and incubated at 37oC for 2 or 3 hours. Growth stopped when the culture reached an optical density (OD) at 650nm between 0.45 and 0.55. In this OD range, the culture is considered to be at a concentration of 108 CFU/mL [Ericksen, B., et al., Antibacterial activity and specificity of the six human {alpha} -defensins. Antimicrob Agents Chemother, 2005. 49 (1): p. 269-75] The bacterial suspension was diluted to $10^6$ CFU/mL in Phosphate Buffer Saline (PBS), which was used as a working culture. The peptides were dissolved in sterile PB to the desired concentration, and 50 $\mu$L was applied to a sterile 96-well polypropylene plate (Eppendorf, Germany). Two-fold serial dilutions of the peptides (in duplicate) were made in PB, and 50 $\mu$L of working culture was applied to each well. The plate was incubated for 2 hours at 37∘C to allow the interaction of the peptides with the bacterial membrane. Subsequently, 100 $\mu$L of doubly concentrated MH medium was added to all wells, and microbial growth was kinetically followed by recording the OD at 650 nm every 5 minutes for 15 hours at 37 °C using a microplate reader (BioTek Instruments, USA). Finally, the growth curves were analyzed and the mathematical processing of the data allowed obtaining a relationship between the peptide concentration and the survival percentage in terms of CFU. By fitting the data to an equation of the type Log [peptide] versus response in OD, it is possible to determine the lethal concentration of 90% of microbial growth (LC$_{90}$) (Table 1). In this way, the average of three individual experiments of the CL$_{90}$ of the bacterial culture for each sequence against strains of multiresistant Gram-negative and Gram-positive microorganisms from clinical isolates is reported.

Table 1. Values expressed in lethal concentration of 90% of the bacterial culture (LC$_{90}$) of SEQ ID NO. 1, SEQ ID NO. 2 and SEQ ID NO. 3 on multi-resistant strains (from the ESKAPE group) from clinical isolates. The values represent the range of concentrations obtained from independent experiments.

| MDR clinical isolated | LC$_{90}$ $\mu$M | | | | | |
|---|---|---|---|---|---|---|
| | Gram-negative | | | | Gram-positive | |
| Peptides | *K. pneumoniae* | *E. coli* | *P. aeruginosa* | *A. baumannii* | *S. aureus* | *E. faecium* |
| SEQ ID NO. 1 | 2-8 | 1-4 | 2-8 | 2-8 | 1-4 | 4-8 |
| SEQ. ID. NO. 2 | 1-4 | 1-4 | 1-4 | 2-8 | 1-4 | 2-8 |
| SEQ. ID. NO. 3 | 2-8 | 1-4 | 1-4 | 4-8 | 2-8 | 4-8 |

Example 3.

Cytotoxicity evaluation

The hemolytic activity of the peptides was evaluated on Wistar rat erythrocytes. The drawn blood was supplemented with heparin in a collecting device (Monovette, Sarstedt, Germany). The blood was centrifuged at 1000 $\times$ g for 10 minutes at 25 °C (Hettich, Germany), and the erythrocyte pellet was washed three successively with sterile saline. Plasma-free erythrocytes were diluted in saline to a final concentration of 4% (v / v). Two-fold serial dilutions of each peptide were mixed with an equal volume of erythrocytes and incubated for 1 hour at 37 oC in U-bottom microplates (Sero-Wel, Bibby Sterilin, UK). PBS and Triton-X 100 at 0.1% (v / v) were used as controls for 0% and 100% hemolysis, respectively. Once the incubation had concluded, the plates were centrifuged at 1000 g for 5 minutes and the supernatant was transferred to flat-bottom polystyrene microplates, where the absorbance was measured at 540 nm, indicative of the release of hemoglobin by the erythrocytes. The percentage of hemolysis (% H) was calculated according to Equation 1, from two independent replications, where H$_{0\%}$ and H$_{100\%}$ correspond to the Abs540nm for the controls with PBS and Triton X-100, respectively, and H$_{peptide}$ at the value of Abs540nm obtained for each peptide concentration.

$$\%H = \frac{H_{peptide} - H_{0\%}}{H_{100\%} - H_{0\%}} \qquad \textit{Equation 1}$$

Similarly, the cytotoxic effect of the peptides represented by SEQ ID NO.1-3 in Hep2 cells was determined. 96-well plates seeded after 48 hours with a completely confluent cell monolayer (1 $\times$ 10$^5$ cells/well) were used. The growth medium was removed from the plates and 100 $\mu$L of peptides dissolved in culture medium without SFBI were added per well at different concentrations from 6.25 $\mu$M to 100 $\mu$M. Six wells were used per concentration of the peptide to be evaluated, and in the remaining wells (6) 100 $\mu$L of culture medium without SFBI were added, the latter constituted the cellular control. The plates were incubated at 37 °C, in a 5% CO$_2$ atmosphere for 72 hours, and were observed daily to determine possible morphological changes in the cell monolayer indicative of cytotoxicity. After this time, cell viability was determined in all wells using the colorimetric assay based on the reduction of the MTT compound by active mitochondrial enzymes in living cells [Mosmann, T., Rapid colorimetric assay for cellular growth and survival: application to proliferation and cytotoxicity assays. J Immunol Methods, 1983. 65 (1-2): p. 55-63] 10 $\mu$L of the MTT solution at a concentration of 5 mg/mL in PBS was added to each well. The cells were incubated again at 37 °C, protected from light, in a 5% CO$_2$ atmosphere for 4 hours. Subsequently, the entire content of the medium was removed from the plate and the supernatant was carefully decanted on filter paper. The precipitate formed was resuspended by adding 100 $\mu$L of anhydrous DMSO to each well, carefully shaking the plate for 5 minutes. The absorbance of the plate was read at 540 nm with a reference filter at 630 nm, in an ELX 808 multi-well plate spectrophotometer (BIOTEK, USA) with the integrated program Gen5 version 2.00.18. The percentage of cell viability associated with each concentration of the peptide was calculated as follows:

$$\% \, cell \, viability = \frac{(Abs_{CT})}{(Abs_{CC})} \, x \, 100 \qquad \textit{Equation 2}$$

AbsCT: mean absorbance value of the cell cultures treated with the peptides. AbsCC: mean absorbance value of the cell controls considered as 100% cell viability. The assay was performed in duplicate for each peptide concentration. The figure shows the two variants of in vitro cytotoxicity assays of the peptides represented by SEQ ID NO. 1, SEQ ID NO. 2 and SEQ. ID. NO. 3.

Example 4.

Evaluation of Time Kill.

This method is based on the determination of the time needed by the peptides represented by SEQ ID NO. 1, SEQ ID NO. 2 and SEQ. ID. NO. 3, and the antibiotics meropenem and ciprofloxacin, to achieve at least a 3-log (1000-fold) decrease in initial bacterial concentration CFUs. Time-dependent death was evaluated against *P. aeruginosa* (ATCC 9027) and *K. pneumoniae* (ATCC 1003) bacterial strains as described in the literature [12], with minor modifications. Working cultures of bacteria (1 × 105 CFU/mL) were exposed to all antimicrobials at the concentration of 2 × the MIC shown for the strain to be tested, in a final volume of 1 ml (v/v). The inoculum in PBS (pH 7.4) without any antimicrobial was considered as a control. After inoculation, all suspensions were incubated at 37 °C. 100 $\mu$l aliquots of the culture were removed at different predetermined times (5, 10, 15, 20, 30, 60, 90, and 120 min), which were diluted 100 × in sterile PBS and spread on Mueller-Hinton agar plates. Plates were incubated for 24 hours at 37 ° C, and cell survival was determined by counting total colonies. CFU versus time curves was constructed for each peptide compound and compared with the reference antibiotics. Bacteria death time is shorter for designed sequences than for conventional antibiotics used as susceptibility control for Gram-negative strains.

Example 5.

Evaluation of the neutralizing activity of LPS by the Monocytes Activation Test (MAT).

This method is based on the determination of pro-inflammatory cytokines released in a whole blood culture induced by agents that generate a pro-inflammatory response. LPS was used as the main inducer of the pro-inflammatory response. To determine the influence of the peptides represented by SEQ ID NO. 1, SEQ ID NO. 2 and SEQ. ID. NO. 3 In the ex vivo system in the presence and/or absence of LPS, the release of IL-6 cytokine was determined by an ELISA. For this, a whole blood culture was incubated in RPMI-1640 medium (Sigma, USA) with a series of dilutions of the peptides covering 0.05-50 nM in the presence and absence of fixed concentrations of LPS at 0.25 EU. / mL (spike). The culture was incubated for 20 h in an Ultima II incubator (Revco Products, USA) at 37 °C with 5% $CO_2$. Subsequently, 50 $\mu$L of the sample was extracted and the cytokine concentration was determined using the standards for each cytokine and capture and biotinylated monoclonal antibodies for each of the cytokines. Co-incubation of a curve of the international LPS standard in the range of 0.05 EU / mL to 2000 EU / mL with whole blood in RPMI was performed on the same plate. The test was performed in triplicate and the means and standard deviation of the values obtained were calculated using the GraphPad Prism v program. 5.0 (GraphPad Software, Inc., USA). To compare the cytokine release values obtained in each test condition (LPS with and without peptides corresponding to SEQ ID NO. 1-3), a multiple student test was used. To compare the effect between the different concentrations of the peptides co-incubated with LPS and the LPS control, a simple classification ANOVA was used followed by Dunnet's multiple comparison test.
The designed sequences show an inhibitory activity of the expression of pro-inflammatory cytokines (IL-6) mediated by LPS in a whole blood culture.

Example 6.

Evaluation of the neutralizing activity of LPS by the LAL PYROCHROME chromogenic assay.

The neutralizing activity of the peptides is represented by SEQ ID NO. 1, SEQ ID NO. 2 and SEQ. ID. NO. 3 on bacterial endotoxins (LPS) using the LAL kinetic chromogenic assay (PYROCHROME®; ACC, USA). All pyrogen-free materials supplied by ACC, USA were used to carry out the test. The test conditions followed the manufacturer's instructions. The peptides to be evaluated were tested in a series of 1:10 dilutions (v / v) from an initial concentration of 20 $\mu$M with a constant endotoxin challenge (LPS) of 0.5 EU / ml. The different peptide concentrations plus the LPS challenge were incubated in a non-pyrogenic microplate (ACC, USA) at 37 °C for 5 minutes. Subsequently, the LAL reagent was added and the reaction kinetics were measured at 450 nm for 1 hour with 15-second intervals at 37 oC in a microplate spectrophotometer. The appearance of a yellow coloration was due to the release of p-nitroaniline from the chromogenic substrate included in the LAL, which is only hydrolyzed with the presence of free LPS in the sample, which activates the enzymatic cascade that leads to hydrolysis of the substrate. Using the KC4 software, the percentages of LPS recovery were obtained, which translates into the amount of free or active LPS that is not neutralized or sequestered by the evaluated peptides.

Example 7.

Evaluation of the antibacterial effects obtained from the combination between different peptide variants represented by SEQ ID NO. 1, SEQ ID NO. 2 and SEQ. ID. NO. 3 on bacterial survival.

Only strains from clinical isolates of Gram-negative bacteria *Klebsiella pneumoniae* and Gram-positive *Staphylococcus aureus* multiresistant to antibiotics were chosen to carry out the combination tests between the peptides. The experiments were carried out according to the methodology of the test described as "checkerboard" or checkerboard [Huang, R.-y., et al., Isobologram Analysis: A Comprehensive Review of Methodology and Current Research. Frontiers in Pharmacology, 2019. 10: p. 1222] This test allows the determination of the fractional inhibitory concentration index (FICI) following Equation 3, which establishes the classification of the effects obtained as follows: synergy (FICI ≤ 0.5), additive (0.5 <FICI ≤ 1), indifferent (1 <FICI ≤ 2) and antagonism (FICI> 2).

$$FICI = \mathbf{FIC}_A + FIC_B = \frac{MIC_{AB}}{MIC_A} + \frac{MIC_{BA}}{MIC_B} \qquad Equation\ 3$$

Where: $MIC_A$: minimum inhibitory concentration of compound A; $MIC_B$: minimum inhibitory concentration of compound B; $MIC_{AB}$: minimum concentration of A that combined with B inhibits growth; $MIC_{BA}$: minimum concentration of B that combined with A inhibits growth. The checkerboard experiment was performed in sterile 96-well polypropylene plates (Eppendorf, Germany). Isolated colonies of each bacterium were used to create $1 \times 10^5$ CFU bacterial cultures in PBS, referred to as a working culture. In addition, 64 $\mu$M stock solutions of the peptides were prepared in PBS. Twofold dilutions in PBS of each peptide were made in 50 $\mu$L final volume. Peptide A was arranged in the columns (2-11) and peptide B in the rows of the plate (B-F). In this way, 60 internal wells of the plate were used for combinations of peptide concentrations between peptide A (0.25-16 $\mu$M) and peptide B (0.075-16 $\mu$M). The first column and the last row corresponded to the concentrations of the uncombined A and B peptides, respectively. As control of bacterial growth, 50 $\mu$L of PBS was added to all the wells of the columns and external rows of the plate. Subsequently, 50 $\mu$L of the working bacterial culture were added to all wells and the plate was incubated at 37°C for 2 h. Finally, 100 $\mu$L of double-concentrated MH broth (MHB) was dispensed to all wells, and microbial growth was followed kinetically by measuring OD at 650 nm for 15 hours at 37 ° C, recording OD values at 5 min intervals on a plate spectrophotometer (BioTek Instruments, USA). Bacterial growth inhibition (MIC) values were taken as values similar to the blanks (controls) of MHB medium incubated in the outermost wells of the plate. The following table shows the mean of the results obtained in three independent experiments.

Table 2. Checkerboard test that checks the synergistic effects that are evidenced with the combinations between the peptide sequences comprised in SEQ. ID: NO. 1, SEQ. ID: NO. 2 and SEQ. ID: NO. 3.

| Bacterial Strain | Combinations A (peptide A)/ B (peptide B.) | $FIC_A$ $(MIC_{AB}/MIC_A)$ | $FIC_B$ μM $(MIC_{BA}/MIC_B)$ | FICIs $(FIC_A+FIC_B)$ | Qualitative Results |
|---|---|---|---|---|---|
| K. pneumoniae | SEQ ID NO. 1 /SEQ ID NO. 2 | 0.25/4.0 | 0.12/1.0 | 0.18 | Synergy |
| | SEQ ID NO. 1 /SEQ ID NO. 3 | 0.25/2.0 | 0.5/4.0 | 0.25 | Synergy |
| | SEQ ID NO. 3/SEQ ID NO. 2 | 0.25/4.0 | 0.12/1.0 | 0.18 | Synergy |
| S. aureus | SEQ ID NO. 1 /SEQ ID NO. 2 | 0.50/4.0 | 0.12/2.0 | 0.18 | Synergy |
| | SEQ ID NO. 1 /SEQ ID NO. 3 | 0.25/2.0 | 0.25/4.0 | 0.18 | Synergy |
| | SEQ ID NO. 3/SEQ ID NO. 2 | 0.25/4.0 | 0.25/2.0 | 0.18 | Synergy |

Example 8

Evaluation of the synergistic effects in the combinations of the peptides represented by SEQ ID NO. 1, SEQ ID NO. 2 and SEQ. ID. NO. 3 with the antibiotic meropenem by expressing the Fractional Inhibitory Concentration Index (FICI).

The checkerboard method was used to evaluate the synergy between Meropenem and the peptides represented by SEQ. ID: NO. 1, SEQ. ID: NO. 2 and SEQ. ID: NO. 3 against multi-resistant bacteria *Klebsiella pneumoneae* and *Staphylococcus aureus* from clinical isolates. The checkerboard method procedure used is similar to that described in Example 7. In this case, meropenem was tested as compound A and the peptides as compound B. The results shown in Table 3 are based on the determination of the Index. fractional inhibitory concentration (FICI) according to the following classification: synergy (FICI ≤ 0.5), additive (0.5 <FICI ≤ 1), indifferent (1 <FICI ≤ 2) and antagonism (FICI> 2). All the hybrid variants produce a synergistic antibacterial effect when combined with Meropenem against the clinical isolates of Gram-negative bacteria *Klebsiella pneumoniae* and Gram-positive multi-resistant *Staphylococcus aureus* to antibiotics (Table 3).

Table 3. Checkerboard test verifying the synergistic effects that are evidenced with the combinations of SEQ. ID: NO. 1, SEQ. ID: NO. 2 and SEQ. ID: NO. 3 and Meropenem (carbapenemic).

| Bacterial strain | Combinations $A_{(MER)}$/ $B_{(SEQ.\,ID:NO.)}$ | $FIC_A$ µg/ml $(MIC_{AB}/MIC_A)$ | $FIC_B$ µM $(MIC_{BA}/MIC_B)$ | FICIs $(FIC_A+FIC_B)$ | Qualitative Results |
|---|---|---|---|---|---|
| K. pneumoniae | MER/SEQ ID NO. 1 | 1.0/8.0 | 0.25/4.0 | 0.18 | Synergy |
| | MER/SEQ ID NO. 2 | 0.5/8.0 | 0.12/2.0 | 0.12 | Synergy |
| | MER /SEQ ID NO. 3 | 1.0/8.0 | 0.12/4.0 | 0.15 | Synergy |
| S. aureus | MER /SEQ ID NO. 1 | 1.0/8.0 | 0.12/2.0 | 0.18 | Synergy |
| | MER /SEQ ID NO. 2 | 0.5/8.0 | 0.12/2.0 | 0.12 | Synergy |
| | MER /SEQ ID NO. 3 | 0.5/8.0 | 0.12/4.0 | 0.09 | Synergy |

**Claims**

1. A peptide with antimicrobial activity **characterized in that** it comprises: an amino acid sequence that has at least 80% identity with an amino acid sequence selected from the group consisting of the sequences SEQ ID NO. 1; SEQ ID NO.2; and SEQ ID NO.3

2. The peptide according to claim 1 further **characterized in that** the cysteines (Cys; C) present in each sequence are forming disulfide bonds and the residue at the terminal carboxyl end is amidated.

3. The peptide according to claim 2 further **characterized in that** Xaa is independently selected from the group consisting of D or L forms of arginine (Arg; R) and lysine (Lys; K); provided that at least four of the Xaa residues are arginine.

4. A combination therapy to a patient with a bacterial infection to control the development of the multi-resistance phenomenon comprising the administration to the patient of at least 2 sequences selected from the group defined in claims 1-3.

5. A combination therapy to a patient with a bacterial infection to control the development of the multi-resistance phenomenon comprising the administration to the patient of a sequence selected from the group defined in claims 1-3 with a beta-lactam antibiotic.

6. The combination therapy according to claim 5 wherein the beta-lactam antibiotic is a carbapenem.

7. A pharmaceutical composition for the treatment and/or prevention of microbial infections comprising:

   a) a therapeutically effective amount of a peptide selected from the group defined in claims 1-3 as active ingredient and
   b) one or more pharmaceutically acceptable excipients.

8. The pharmaceutical composition according to claim 7 further comprises a second peptide selected from the group defined in claims 1-3 as an active ingredient.

9. The pharmaceutical composition according to claim 7 further comprises a beta-lactam antibiotic as an active ingredient.

10. The pharmaceutical composition according to claim 9 wherein the beta-lactam antibiotic is meropenem.

11. Use of a peptide selected from the group described in claims 1-3, of a combination therapy selected from those described in claims 4-6, or of a pharmaceutical composition selected from those described in claims 7-10 characterized because said use is for the manufacture of a medicament for the prevention and/or treatment of microbial infections.

12. The use according to claim 11 where the infection is caused by a pathogenic strain of Gram-negative bacteria or Gram-positive bacteria belonging to the ESKAPE group.

13. The use according to claim 12, wherein the Gram-negative bacteria show multi-resistance to conventional antibiotics and are selected from the group comprising *Klebsiella pneumoneae, Acinetobacter baumannii, Pseudomona aeruginosa,* and *Escherichia coli.*

14. The use according to claim 12, wherein the Gram-positive bacteria show multi-resistance to conventional antibiotics and are selected from the group comprising *Enterococcus faecium* or *Staphylococcus aureus.*

15. A method of treatment or prevention a microbial infection comprising the administration of a peptide selected from the group described in claims 1-3, of a combination selected from those described in claims 4-6, or of a pharmaceutical composition selected from those described in claims 7-10 in a therapeutically effective amount to a subject with a microbial infection.

16. The method of treatment according to claim 15 wherein the infection is caused by a pathogenic strain of Gram-negative bacteria or Gram-positive bacteria belonging to the ESKAPE group.

17. The method of treatment according to claim 16, wherein the Gram-negative bacteria show multi-resistance to conventional antibiotics and are selected from the group comprising *Klebsiella pneumoneae, Acinetobacter baumannii, Pseudomona aeruginosa,* and *Escherichia coli.*

18. A method of treatment according to claim 16, wherein the Gram-positive bacteria show multi-resistance to conventional antibiotics and are selected from the group comprising *Enterococcus faecium* or *Staphylococcus aureus.*

# Peptide Sec.1
**Area % Report**

Data File: D:\Lab Analisis\Servicio RP HPLC\Data\Año 2017\M17P31\M17P31 003 9-14-2017 2-47-43 AM.dat
Method: C:\EZChrom Elite\Enterprise\Projects\Default\Method\untitled.met
Acquired: 9/14/2017 2:59:08 AM
Printed: 3/2/2020 4:24:21 PM

## K-2501 Results

| Retention Time | Area | Area % | Height | Height % |
|---|---|---|---|---|
| 13.350 | 24930070 | 98.47 | 1452995 | 96.71 |
| 13.767 | 387247 | 1.53 | 49445 | 3.29 |

| Totals | | | | |
|---|---|---|---|---|
| | 25317317 | 100.00 | 1502440 | 100.00 |

M17P31ss
Average Mass = 3262.6932, Monoisotopic Mass = 3260.5733
N-Terminus = H, C-Terminus = NH2

1    YCPYG NRLNY WGRAR GHIGT KSCRR SY

## Peptide Sec.2

**Area % Report**

Data File: D:\Lab Analisis\Servicio RP_HPLC\Data\Año 2017\M17P32\M17P32 008 9-18-2017 1-20-59 PM.dat

Method: C:\EZChrom Elite\Enterprise\Projects\Default\Method\untitled.met

Acquired: 9/18/2017 1:32:27 PM

Printed: 3/2/2020 4:26:45 PM

**K-2501 Results**

| Retention Time | Area | Area % | Height | Height % |
|---|---|---|---|---|
| 15.550 | 46269 | 1.54 | 6127 | 2.22 |
| 15.800 | 2882982 | 95.95 | 257857 | 93.39 |
| 16.100 | 75424 | 2.51 | 12114 | 4.39 |

| | Totals | | | |
|---|---|---|---|---|
| | 3004675 | 100.00 | 276098 | 100.00 |

M17P32ss

Average Mass = 3368.8520, Monoisotopic Mass = 3366.6502

N-Terminus = H, C-Terminus = NH2

1    YSTSC RYFRL DYRSF SVGTK SCVRR GIY

<Sample Information>

| | | | |
|---|---|---|---|
| Sample Name | : Y19P94ss C2F4 | | |
| Sample ID | : Y19P94ss C2F4 | | |
| Data Filename | : Y19P94ss C2F4.lcd | | |
| Method Filename | : Gradient de 5 a 60% B en 35min equil final.lcm | | |
| Batch Filename | : Y19P94ss C2.lcb | | |
| Vial # | : 1-7 | Sample Type | : Unknown |
| Injection Volume | : 150 uL | | |
| Date Acquired | : 5/21/2020 9:37:06 PM | Acquired by | : System Administrator |
| Date Processed | : 5/25/2020 8:13:35 AM | Processed by | : System Administrator |

<Chromatogram>

<Peak Table>

Detector A 226nm

| Peak# | Ret. Time | Area | Height | Conc. | Unit | Mark | Name |
|---|---|---|---|---|---|---|---|
| 1 | 16.638 | 374561 | 38076 | 4.375 | | M | |
| 2 | 16.818 | 8154501 | 892242 | 95.239 | | V M | |
| 3 | 17.063 | 33080 | 12338 | 0.386 | | V M | |
| Total | | 8562143 | 942655 | | | | |

Y19P94ss

Average Mass = 3442.9462, Monoisotopic Mass = 3440.7108

N-Terminus = H, C-Terminus = NH2

1    YSTSC RYFRL NHWSR ARGHI SCVRR GIY

Figure 1.

Figure 2.

Figure 3.

Figure 4.

Figure 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013113296 A

### Non-patent literature cited in the description

- **MULANI, MS et al.** Emerging Strategies to Combat ESKAPE Pathogens in the Era of Antimicrobial Resistance: A Review. *Frontiers in microbiology,* 2019, vol. 10, 539-539
- **MOURTADO, R. et al.** Design of stapled antimicrobial peptides that are stable, non-toxic and kill antibiotic-resistant bacteria in mice. *Nature Biotechnology,* 2019, vol. 37 (10), 1186-1197
- **HALE, J.D. ; R.E. HANCOCK.** Alternative mechanisms of action of cationic antimicrobial peptides on bacteria. *Expert Rev Anti Infect Ther,* 2007, vol. 5 (6), 951-9
- **MONTERO-ALEJO, V. et al.** Defensin-like peptide from Panulirus argus relates structurally with beta-defensin from vertebrates. *Fish Shellfish Immunol,* 2012, vol. 33 (4), 872-9
- **MONTERO-ALEJO, V. et al.** Panusin represents a new family of beta-defensin-like peptides in invertebrates. *Dev Comp Immunol,* 2017, vol. 67, 310-321
- **PERDOMO-MORALES, R. et al.** The trypsin inhibitor panulirin regulates the prophenoloxidase-activating system in the spiny lobster Panulirus argus. *J Biol Chem,* 2013, vol. 288 (44), 31867-79
- **PERDOMO, M.R. et al.** *Composition from lobster hemocyte extract for detection of lipopolysaccharides, peptidoglycans, and 1,3-beta-d-glucans,* 2014
- **KAISER, E. et al.** Color test for detection of free terminal amino groups in the solid-phase synthesis of peptides. *Anal Biochem,* 1970, vol. 34 (2), 595-8
- **GONZALEZ, J. et al.** Effect of the position of a basic amino acid on C-terminal rearrangement of protonated peptides upon collision-induced dissociation. *J Mass Spectrom,* 1996, vol. 31 (2), 150-8
- **ERICKSEN, B. et al.** Antibacterial activity and specificity of the six human {alpha} -defensins. *Antimicrob Agents Chemother,* 2005, vol. 49 (1), 269-75
- **MOSMANN, T.** Rapid colorimetric assay for cellular growth and survival: application to proliferation and cytotoxicity assays. *J Immunol Methods,* 1983, vol. 65 (1-2), 55-63
- **HUANG, R.-Y. et al.** Isobologram Analysis: A Comprehensive Review of Methodology and Current Research. *Frontiers in Pharmacology,* 2019, vol. 10, 1222